# EUROPEAN PATENT SPECIFICATION

(11) **EP 3 895 546 B1**
(45) Date of publication and mention of the grant of the patent: **28.05.2025**
(21) Application number: 21168161.4
(22) Date of filing: 13.04.2021
(51) Int. Cl.: A23L 2/48, A23B 5/01, A23C 3/07, A23L 3/005, A61L 2/00, A61L 12/06

(54) **METHOD FOR REDUCING THE MICROBIAL LOAD OF A LIQUID AND RELATED APPARATUS FOR CARRYING IT OUT**
VERFAHREN ZUR VERMINDERUNG DER MIKROBIELLEN BELASTUNG EINER FLÜSSIGKEIT UND ZUGEHÖRIGE VORRICHTUNG ZUR DURCHFÜHRUNG DESSELBEN
PROCÉDÉ DE RÉDUCTION DE LA CHARGE MICROBIENNE D'UN LIQUIDE ET APPAREIL ASSOCIÉ POUR SA MISE EN OEUVRE

(30) Priority: 14.04.2020 IT 202000007867
(43) Date of publication of application: 20.10.2021
(73) Proprietor: WAXY S.R.L. Societa' Benefit, 15121 Alessandria (AL) PRESSO ENNE3 (IT)
(72) Inventor: FONTANA, Mauro, 10045 Piossasco (TO) (IT); BISOTTI, Stefano, 29121 Piacenza (IT); COLOMBO, Valter, 20019 Bresso (MI) (IT); BRANCIAROLI, Osvaldo, 10100 Torino (IT)
(74) Representative: Bottero, Carlo

(56) References cited:
- CH-A5- 563 805
- FR-A1- 2 539 997
- RU-C1- 2 083 140
- RU-C1- 2 415 595
- US-A- 3 934 042

## Description

### Field of the invention

The present invention relates to a method for reducing the microbial load of a liquid and a relative apparatus for carrying out said method.

The present invention is suitable for handling different types of liquids, including liquids containing gases, solids and/or liquid substances, such as mixtures, slurries, gels, etc. The method according to the present invention can be applied to substantially any material or mixture of materials, in a liquid state and having viscosities in a wide range of values, capable of flowing.

### Background of the Invention

As it is known, in many industrial sectors, such as food, cosmetics, pharmaceuticals and waste disposal, there is the need to subject different types of liquids to treatments capable of reducing the microbial load present in them.

For example, food liquids intended for human and animal consumption, such as beverages (e.g. water, milk, fruit juices, wine), oil, canned food, animal feed, etc., require treatments capable of preventing the proliferation of contaminating microorganisms, which find a favourable environment for their growth in these products. Uncontrolled proliferation of microorganisms can compromise the safety and shelf life of products.

Similarly, the need to obtain liquids with a controlled microbial load is also felt in the processes for the production of cosmetic products (creams, lotions, etc..), pharmaceutical products (active ingredients, pharmaceutical intermediates, etc..) or in the processes for the treatment of liquid waste such as sewage produced by civil settlements, wastewater from industrial processes, sewage sludge, biological liquid waste (blood, urine, etc..).

The control of the microbial load in the aforesaid liquids meets the need to ensure the safety of their consumers or users and the safety of the working environments in which they are used.

Known methods in the state of the art for reducing the microbial load of a liquid include, for example, sterilization, pasteurization, ozonation, microfiltration treatments, and others. These treatments, although effective, nevertheless have several disadvantages, such as high energy consumption, particularly in the case of methods involving heat treatment of liquids, the use of chemicals, the use of bulky equipment or equipment operating with high pressure fluids (e.g. boilers for the production of steam), the use of expensive filtration membranes, etc..

In the state of the art, methods for reducing the microbial load of food products are also known, which involve treating these products by irradiation with electromagnetic radiation.

EP 2060186 A1 describes, for example, a treatment for sanitising the rind of Gorgonzola PDO cheese in which the rind is subjected to a series of successive heat treatments in which: the first heat treatment is carried out at low temperature (0-2°C) for a duration of 0.5 - 4 hours in a forced air cooling tunnel; the second heat treatment is carried out at high temperature (80-85°C) by irradiation through infrared radiation for a duration of 5 - 120 seconds; the third heat treatment is carried out at low temperature (0-2°C) for a duration of 0.5 - 4 hours in a forced air cooling tunnel. This treatment method is described for exclusive use in cheese sanitization; the overall duration of the treatments is rather long, about 1-8 hours.

A method for reducing the microbial load applied to sausages is described in EP 2594136 A1. The method involves a low temperature heat treatment (0-2°C) followed by a high temperature heat treatment (72-80°C) performed through irradiation with infrared radiation and by a second low temperature heat treatment (1-4°C).

A method for reducing the microbial load using electromagnetic radiations applied to eggs is described in EP 3491933 A1. The method includes a first heat treatment at a temperature within the range 70-82°C followed by a second heat treatment at a temperature within the range 85-105°C and by a cooling phase at a temperature within the range 15-25°C. The two heat treatment steps are performed through irradiation with infrared radiation having two different wavelengths (λ = 5-18 micrometers for the first treatment and λ = 18-30 micrometers for the second treatment).

CH563805A5 refers to a process for the transfer of electromagnetic energy in colloidal, micellar or molecular media, characterized in that the colloidal, micellar or molecular medium to be treated is brought to the treatment temperature, away from air, then subjected to irradiation using electromagnetic waves during the flow of this medium and by the fact that the colloidal, micellar or molecular medium to be treated is maintained under pressure for the duration of this irradiation, so that the Brownian motion generated within this medium is activated and the cross section of the molecules and micellar granules is increased.

US3934042A discloses a method and an apparatus for the irradiative treatment of beverages to sterilize or pasteurize them, in which the beverage is pumped through the system out of contact with the air. The entering beverage is heat exchanged against the exiting beverage, and is then subjected to ultra-violet irradiation, and further heat exchange against the exiting beverage.

The known methods described above, although they effectively reduce the microbial load and limit energy consumption compared to the methods used in the food sector (e.g. sterilization and pasteurization), have the disadvantage of being usable only in the case of substantially solid products, such as sausages, cheese and eggs.

### Summary of the invention

In view of the aforementioned state of the art, the Applicant has set itself the primary objective of providing a method and apparatus for reducing the microbial load in a liquid, which overcome at least in part the drawbacks of the methods known in the state of the art.

In particular, a specific object of the present invention is to provide a method and an apparatus for reducing the microbial load in a liquid that are quick and easy to carry out and characterized by low energy consumption.

A second object of the present invention is to provide a method and an apparatus for reducing the microbial load in a liquid that are applicable to liquids having a variety of chemical, physical and/or rheological characteristics.

A third object of the present invention is to provide a method and an apparatus that allow to reduce the microbial load in a liquid without significantly altering the other characteristics of the liquid being treated; for example, in the case of food liquids, without significantly altering the organoleptic qualities.

A further object of the present invention is to provide a method and an apparatus for reducing the microbial load in a liquid, which can be readily integrated into existing manufacturing processes that produce or employ such liquids.

A further object of the present invention is to provide a method and an apparatus for reducing the microbial load in a liquid that can be adapted for use in treating small and large volumes of liquids.

The Applicant has found that the aforementioned and other objects, which will be more fully explained in the description below, can be achieved by exposing a liquid whose microbial load is to be reduced to infrared radiation (IR) emitted according to a specific irradiation sequence in which periods of exposure of the liquid to infrared radiation (heating phases) alternate with periods in which the liquid is not exposed - periods of non-exposure - to the aforesaid radiation (cooling phases) .

It has been surprisingly found, in fact, that the aforesaid irradiation sequence is able to devitalize the microorganisms present in the liquid, thus reducing the microbial load in a simple, rapid way and with a very low energy consumption.

Without referring to any particular theory, it is believed that the exposure of the liquid to infrared radiation induces a sudden local heating of the liquid, which is followed by an equally sudden cooling of the same when the exposure to infrared radiation ceases. An irradiation sequence comprising at least two heating phases spaced out with a cooling phase thus generates a thermal shock in the body of the liquid, which devitalizes the microorganisms present in it; since the energy transmitted to the liquid in the form of radiation is relatively low and the duration of the treatment is relatively short, the irradiation does not significantly modify the other characteristics of the liquid, such as chemical composition, rheological characteristics and organoleptic properties.

It has also been observed that a relatively small number (less than 10) of short periods of exposure to IR radiation (of up to 15 seconds each) are sufficient to reduce the microbial load in treated liquids to the levels required in most industrial applications, particularly in the case of the treatment of food and cosmetic liquids. The treatment is therefore effective, quick and can be used to treat a wide variety of different liquids.

In accordance with the present invention, the irradiation of the liquid with infrared radiation is carried out while the liquid is made to flow inside a tubular conduit, which is structured to be transversely crossed by the infrared radiation emitted by a plurality of IR sources placed in the vicinity thereof and suitably spaced between them along the direction of flow of the liquid in the conduit.

By using IR sources extending for a suitable length measured along the direction of flow of the liquid in the conduit, and by suitably spacing each IR source from the next one, it is possible to subject the liquid to a desired irradiation sequence, exposing the liquid during its path in the tubular conduit to alternating heating and cooling phases, both of predetermined duration for a given velocity of flow of the liquid. For a given configuration of the apparatus (arrangement, length and reciprocal distance of the sources), it is also possible to adjust the exposure and non-exposure times of the liquid to the IR radiation (i.e. the duration of the heating and cooling phases) by varying the velocity of flow of the liquid in the conduit.

Since no high-temperature heat treatments are performed and no chemical additives are used, the method for reducing the microbial load with infrared radiation described herein is characterized by low energy consumption and reduced environmental impact. The method based on irradiation with infrared radiation also allows a homogeneous treatment of the liquid, thus ensuring the reproducibility of the obtainable effect of reduction of the microbial load.

The method and the apparatus, moreover, have a high flexibility of use, being able to be used for the treatment of liquids having also very different chemical composition and rheological characteristics.

A further advantage of the present invention lies in the fact that the treatment method is carried out by means of an apparatus comprising one or more modular elements (treatment modules), each of which is capable of treating a certain mass of liquid in the unit of time. Therefore, by varying the number of treatment modules of the apparatus and by connecting them in parallel or in series, it is possible to treat masses of liquid that may vary greatly based on the needs of the user.

Furthermore, the method and the apparatus according to the present invention can be easily integrated into existing industrial plants by connecting them, for example, to plants for preparing and/or packaging the liquids of interest, even in confined spaces taking into consideration the small overall dimensions of the treatment modules. The invention is defined in the appended claims.

In accordance with a first aspect, therefore, the present invention relates to a method for reducing the microbial load of a liquid comprising the phases:
a. providing at least one treatment module (1) comprising:
   - at least one tubular conduit (12) having at least one portion (14) transparent to infrared radiation;
   - three sources (16a, 16b, 16c) of infrared radiation located in the vicinity of said portion (14) transparent to infrared radiation, said at least two sources (16a, 16b, 16c) being positioned along a direction parallel to the longitudinal axis of said tubular conduit (12), at a distance r from each other, and being configured to transmit at least one infrared radiation (18);
b. making the liquid (20) flow into said tubular conduit (12);
c. exposing the liquid (20) while it flows inside the tubular conduit (12) to the infrared radiation (18) emitted by said at least three sources (16a, 16b, 16c) wherein the liquid flowing inside the tubular conduit is treated according to an irradiation sequence comprising: i) first exposure of the liquid to infrared radiation for a time within the range 0.1 - 5 seconds, which is a first heating phase; ii) second exposure of the liquid to infrared radiation for a time within the range 0.2 - 10 seconds, which is a second heating phase; iii) third exposure of the liquid to infrared radiation for a time within the range 1.5 - 15 seconds, which is a third heating phase; wherein said phases i), ii) and iii) are interspersed with periods of non-exposure of the liquid to infrared radiation of duration within the range 0.1 - 7 seconds, which are cooling phases.

In accordance with a second aspect, the present invention relates to an apparatus (100) for carrying out the aforesaid method for reducing the microbial load in a liquid comprising at least one treatment module (1) comprising:
- at least one tubular conduit (12) having at least one portion (14) transparent to infrared radiation;
- three sources (16a, 16b, 16c) of infrared radiation located in the vicinity of said portion (14) transparent to infrared radiation, said three sources (16a, 16b, 16c) being positioned along a direction parallel to the longitudinal axis of said tubular conduit (12), at a distance r from each other, and being configured to transmit at least one infrared radiation (18) on a liquid (20) flowing inside said tubular conduit (12) wherein said distance r is within the range 1 cm - 50 cm; wherein each of said sources (16a, 16b, 16c) extends for a length (L, L', L"), measured parallel to the longitudinal axis of said tubular conduit (12), within the range 10 cm - 100 cm.

Further characteristics of the process according to the present invention are defined in the dependent claims 2 - 6.

For the purposes of the present invention, the term infrared radiation means electromagnetic radiation having a wavelength comprised within the range 0.7 - 1,000 micrometers.

### Description of the figures

The features and advantages of the process according to the present invention will be more apparent from the following description referring to the appended figures, wherein:
- figure 1, which is a schematic representation of a first embodiment of the present invention;
- figure 2, which is a schematic representation of a second embodiment of the present invention.

### Detailed description of the invention

The following description and the following examples of embodiment are provided for the sole purpose of illustrating the present invention and are not to be understood in a sense limiting the scope of protection defined by the appended claims.

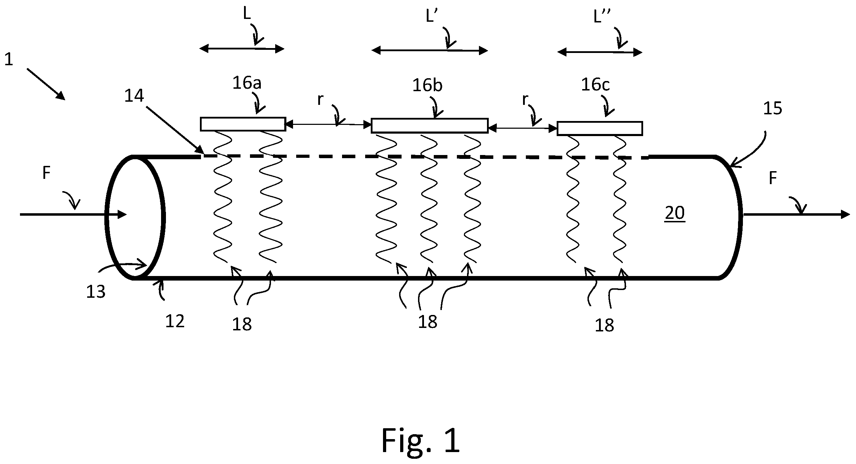

With reference to Figure 1, a treatment module 1 according to the invention comprises at least a tubular conduit 12, the inner surface of which defines a flow cavity into which the liquid to be treated can flow. The tubular conduit 12 comprises an inlet opening 13 and an outlet opening 15 defined by the inner surface of the conduit 12, through which the liquid 20 enters and exits.

At least a portion 14 of the conduit 12 is transparent to infrared radiation; in particular, the aforesaid portion 14 is transparent to at least infrared radiation having a wavelength within the range 1 - 100 micrometers, preferably within the range 20 - 75 micrometers, more preferably within the range 40 - 60 micrometers.

For the purposes of the present invention, the expression "portion transparent to infrared radiation" means that said portion allows the passage of the infrared radiation having a wavelength and intensity such as to allow the reduction of the bacterial load of the liquid flowing in the tubular conduit so as to achieve the microbial load reduction effect illustrated in the present description.

The portion 14 that is transparent to infrared radiation can be made from materials known to the expert in the field, such as quartz, Vycor^{®}, glass ceramic.

In a preferred embodiment, the aforesaid material is quartz. Preferably, quartz has one or more of the following characteristics:
- density within the range 2.15 - 2.21x10³ kg/m³;
- hardness KHN₁₀₀ within the range 550 - 580;
- compressive strength within the range 1.0 - 1.2 x 10⁹ Pa;
- tensile strength within the range 4.7 - 5.0 x 10⁷ Pa (N/m²).

The material known by the trade name Vycor^{®}, manufactured by Corning Glass Works, is a high-temperature, silica glass having a very high thermal shock resistance, consisting of about 96% silica and about 4% boron trioxide.

In a preferred embodiment, the entire tubular conduit 12 is made of a material transparent to infrared radiation, such as the aforesaid quartz.

The profile of the tubular conduit 12 may be substantially of any shape, e.g. circular, square, rectangular, etc. Preferably, the tubular conduit 12 has a circular profile.

The treatment module 1 comprises three sources 16a, 16b, 16c of infrared radiation located in the vicinity of said portion 14 transparent to infrared radiation, external to the tubular conduit 12.

The sources 16a, 16b, 16c are positioned along a direction parallel to the longitudinal axis of said tubular conduit 12, at a distance r (greater than zero) from each other. The distance r is within the range 1 cm - 50 cm, preferably within the range 5 cm - 30 cm.

The sources 16a, 16b, 16c may be separated by the same distance r from each other. The sources 16a, 16b, 16c, however, may also be positioned at different distances r from each other.

In an embodiment, the module 1 comprises a succession of n sources 16a, 16b, 16c of infrared radiation arranged along a direction parallel to the longitudinal axis, each spaced from the next one by a distance r, where n is an integer within the range 2-10, preferably within the range 2-5. According to the invention, n equals 3.

For the purposes of the present invention, infrared radiation sources of the type known in the state of the art and commercially available may be used. Preferably, the infrared radiation sources comprise a filament source.

Preferably, the power of each source of infrared radiation is within the range 1,000 - 4000 W.

Preferably, in the various embodiments of the present invention, the sources 16a, 16b, 16c develop predominantly linearly in a direction parallel to the longitudinal axis of said tubular conduit 12.

According to the invention, each IR source 16a, 16b, 16c extends linearly in a direction parallel to the longitudinal axis of the tubular conduit 12, for a length L, L', L", measured parallel to the longitudinal axis of said tubular conduit 12, within the range 10 cm - 100 cm, preferably within the range 15 cm - 80 cm, even more preferably within the range 20 cm - 60 cm. Each source may have a length L, L', L" equal to or different from the length of the other sources present.

In an embodiment, the treatment module is housed within a refractory material support, which completely encloses it (not shown in the figure).

Each source 16a, 16b, 16c is configured to transmit an infrared radiation 18 preferably having a wavelength within the range 1 - 100 micrometers, more preferably within the range 20 - 75 micrometers, even more preferably within the range 40 - 60 micrometers, to the liquid 20 flowing inside the tubular conduit 12.

In carrying out the treatment method, the liquid 20 is made to flow into the conduit 12, for example in the direction indicated by the arrow F, at a desired flow velocity v, by means of pumping means, either by push or suction (not shown in the figure), or by gravitational fall. At the source 16a, the mass of the liquid 20 is exposed to the infrared radiation 20 emitted by said source.

The emission intensity of the radiation from each source 16a, 16b and 16c is preferably adjusted such that the radiation 18 penetrates the mass of the liquid 20 until it impacts against the wall of the tubular conduit 12 opposite the portion 14 transparent to the infrared radiation. This can be done by adjusting the emission power of the source, taking into account the density of the treated liquid. Typically, the applied power required for this purpose decreases as the density of the liquid to be treated increases.

The exposure of said mass of the liquid 20 to the infrared radiation of the source 16a lasts until said mass has travelled the entire length L of extension of the source 16a (heating phase). In the section of length r separating the source 16a from the source 16b, the aforesaid mass of the liquid 20 is not exposed to any infrared radiation, thus the cooling of the liquid 20 is carried out (cooling phase).

Similarly to what happens at the source 16a, the mass of the liquid 20 is subjected to a second phase of exposure to IR radiation at the source 16b. The duration of this second heating phase is determined by the length extension L' of the source 16b along the flow direction F and by the flow velocity v of the liquid 20. This second phase of heating the liquid is followed by a second phase of cooling the liquid at the section of conduit 12 of length r, which separates the source 16b from the source 16c, in which the liquid 20 is not exposed to the infrared radiation.

In the embodiment exemplified in Figure 1, in its path across the conduit 12, the liquid 20 is treated with the infrared radiation at the source 16c (optional) in a manner similar to that described for the sources 16a and 16b. At the end of this third heating phase, the liquid 20 outflows from the opening 15 of the tubular conduit 12.

The liquid 20 exiting the tubular conduit 12 may be directed to other processes or accumulated in storage tanks. For example, the treated liquid can be stored at room temperature or in a refrigerated environment (e.g. 4 - 10°C), or passed through a heat exchanger or blast chiller.

According to the invention, the liquid flowing inside the tubular conduit is treated according to an irradiation sequence comprising:
i) first exposure of the liquid to infrared radiation for a time within the range 0.1 - 5 seconds, which is a first heating phase;
ii) second exposure of the liquid to infrared radiation for a time within the range 0.2 - 10, which is a second heating phase;
iii) third exposure of the liquid to infrared radiation for a time within the range 1.5 - 15, which is a third heating phase;
wherein the aforesaid phases i), ii) and iii) are interspersed with periods of non-exposure of the liquid to infrared radiation of duration within the range 0.1 - 7, which are cooling phases.

The liquid fed into the tubular conduit inlet is preferably at a temperature within the range 4°C - 25°C.

The treatment of the liquid in accordance with the method according to the present invention may advantageously be carried out in a tubular conduit 12 having a flow cavity with a diameter within the range 0.1 cm - 2.0 cm, preferably within the range 0.2 cm - 1.2 cm.

Preferably, the tubular conduit 12 has an overall length within the range 50 cm - 300 cm, preferably within the range 100 cm - 200 cm. The desired irradiation sequence can also be achieved by connecting two or more treatment modules in series.

The tubular conduit is preferably linear in shape, but may also have a curved shape (e.g. coil).

The flow of the liquid can be horizontal, vertical or inclined.

Preferably, the volumetric flow rate of the liquid flowing in the tubular conduit is within the range 0.1 1/min - 10 l/min, more preferably within the range 1.0 1/min - 5.0 l/min.

The aforesaid dimensions of the tubular conduit and the volumetric flow rate values allow to carry out the treatment for abating the microbial load in the liquid, maximizing the energy efficiency.

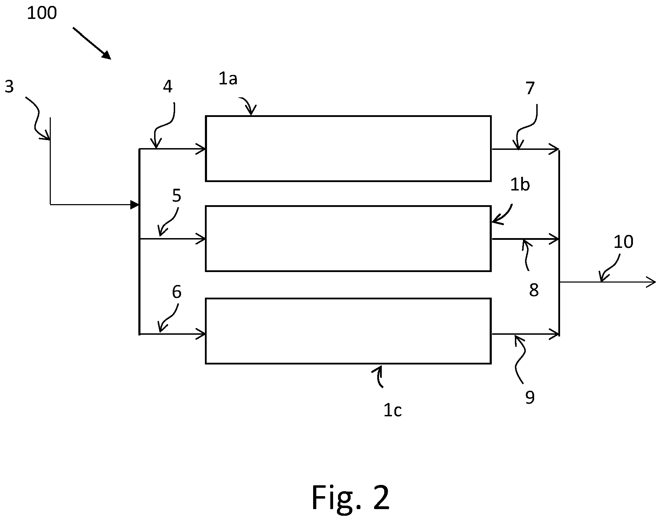

Since it is preferable not to use tubular conduits whose diameter sizes exceed those of the aforesaid ranges, if it is desired to carry out the treatment of relatively large masses of liquid, it is preferable to use a plurality of treatment modules of the type described above, connected together in parallel, as shown schematically in figure 2.

In Figure 2, an embodiment of an apparatus 100 comprising three treatment modules 1a, 1b, 1c connected to each other in parallel is illustrated. A flow 3 of a liquid to be treated is fed to the apparatus 100, which is divided into three aliquots 4, 5, 6, each of which is sent to one of the treatment modules 1a, 1b, 1c. The treated liquids 7, 8, 9 exiting from the treatment modules 1a, 1b, 1c, respectively, are merged into a single final flow 10, which is fed to possible further thermal treatments or to storage.

The aforesaid liquids are moved in the apparatus 100 by, for example, pumping systems not shown in Figure 2.

The use of treatment modules assembled in parallel in a single apparatus offers the advantage of making the method according to the present invention easily sizable according to the volumes of liquid to be treated. As mentioned above, the treatment modules may also be connected in series, for example in the event that it is desired to treat the liquid along a flow path greater than that defined by a single treatment module.

For example, the apparatus may comprise a number of treatment modules connected in parallel within the range 2-100, preferably within the range 2-50, more preferably within the range 2-20.

The method and the apparatus according to the present invention can be used to reduce the microbial load of different types of liquids. Treatable liquids include liquids containing gases, solids and/or liquid substances. They may have higher or lower viscosities and therefore also take the form of mixtures, slurries, emulsions, gels and the like.

Preferably, the liquid is selected from: food liquid, for human and animal consumption (e.g. water, milk, oil, wine, fruit juices, canned food, jam, etc.); cosmetic liquid (e.g. cream, lotion, emulsions, gels, foams, oils, soaps, perfumes, toothpaste, etc.); biological liquid (e.g. blood, urine); hydrocarbon liquid (e.g. lubricants, coolants, fuels, oil drilling sludge, etc.); liquid waste (civil or industrial waste water, sewage, sludge from water purification plants, etc.), heat transfer liquid (e.g. cooling and heating liquids for boilers, evaporative towers, heat exchangers, etc.).

Preferably, the treated liquid has a viscosity at 20°C within the range 0.5 mPa·s - 1*10⁶ mPa-s (measured with Brookfield viscometer).

The treatment with infrared radiation according to the present invention makes it possible to reduce the microbial load present in a liquid due to the presence of microorganisms such as, for example, bacteria, viruses, yeasts and moulds. Examples of microorganisms are: *Enterobacteriaceae, Escherichia coli, Salmonella* Spp., *Bacillus cereus, Staphylococcus aureus* and *Listeria* Spp..;.

To further illustrate the features of the present invention, the following example embodiment is provided, which should be understood as illustrating the present invention and not in a sense limiting the scope of protection defined by the appended claims.

### EXAMPLE

The effectiveness of the present invention was tested on samples of a liquid food consisting of reconstituted skimmed milk powder having a dry residue of about 15 g/100 g of product. To this end, a treatment module was set up consisting of a tubular conduit made entirely of quartz transparent to infrared radiation, having a diameter of 0.5 cm and a length of 140 cm. A system of irradiation of infrared radiations was placed on the outer circumference of the tube, comprising 3 IR filament sources (each developing linearly for a length equal to 40 cm in a direction parallel to the longitudinal axis of the tubular conduit and having a power equal to 1,000 W) arranged along the axial direction of the tubular conduit at a distance r equal to 10 cm. The source was set to radiate towards the flow cavity of the tubular conduit an IR radiation of wavelength 47 - 49 micrometers.

The liquid was made to flow into the module at a flow rate of 2 l/min.The total volume of liquid treated was 120 litres.

Under the aforesaid conditions, the total duration of the treatment, corresponding to the time taken for the liquid advancing front to cross the entire length of the tubular conduit, was 2.4 seconds.

The effectiveness of the treatment in reducing the microbial load in the liquid was assessed by comparing the results of microbiological analyses conducted on a sample of the liquid before and after the treatment. The results of the analyses and the methods of analysis used are given in Table 1 below, where the extent of reduction in microbial load is expressed in terms of logarithmic reduction per gram of sample (logarithm of the ratio of the number of initial microorganisms to the number of microorganisms after the treatment).

**Table 1**

| Microorganisms | Abatement log | Method |
|---|---|---|
| Enterobacteriaceae/g | 4 | AFNOR No. 3M 01/8-06-2001 |
| E. coli/g | 3 | AFNOR No. 3M 01/8-06-2001 |
| TVC (*total viable count*) at 32°C | 4 | UNI EN ISO 4833:2004 |
| Salmonella Spp/g | 3 | UNI EN ISO 6579:2008 |
| Moulds and yeasts/g | 3 | ISTISAN 96-35 |
| Bacillus cereus/g | 3 | ISTISAN 96-35 |
| S. aureus/g | 3 | UNI EN ISO 6888-1:2004 |
| Listeria spp./g | 3 | UNI EN ISO 11290-2:2005 |

No significant differences were shown in the product before and after the treatment according to the invention in relation to the rheological (viscosity), organoleptic and colour characteristics.

Similar results in terms of reduction of the microbial load have been obtained on the following liquids: water, saturated aqueous saline solution, skimmed milk, raw whole milk, milk cream with a fat content of 40%, milk cream with a fat content of 60%, liquid mascarpone preparation, fruit purees, liquid egg-based preparations, edible oils, cosmetic creams.

## Claims

1. Method for reducing the microbial load of a liquid comprising the following phases:
a. providing at least one treatment module (1) comprising:
- at least one tubular conduit (12) having at least one portion (14) transparent to infrared radiation;
- three sources (16a, 16b, 16c) of infrared radiation located in the vicinity of said portion (14) transparent to infrared radiation, said three sources (16a, 16b, 16c) being positioned along a direction parallel to the longitudinal axis of said tubular conduit (12), at a distance r from each other, and being configured to transmit at least one infrared radiation (18);
b. making the liquid (20) flow into said tubular conduit (12);
c. exposing the liquid (20) while it flows inside the tubular conduit (12) to the infrared radiation (18) emitted by said three sources (16a, 16b, 16c);
wherein the liquid flowing inside the tubular conduit is treated according to an irradiation sequence comprising:
i) first exposure of the liquid to infrared radiation for a time within the range 0.1 - 5 seconds, which is a first heating phase;
ii) second exposure of the liquid to infrared radiation for a time within the range 0.2 - 10 seconds, which is a second heating phase;
iii) third exposure of the liquid to infrared radiation for a time within the range 1.5 - 15 seconds, which is a third heating phase;
wherein said phases i), ii) and iii) are interspersed with periods of non-exposure of the liquid to infrared radiation of duration within the range 0.1 - 7 seconds. seconds, which are cooling phases.

2. Method according to claim 1, wherein said three sources (16a, 16b, 16c) develop predominantly linearly in a direction parallel to the longitudinal axis of said tubular conduit (12).

3. Method according to claim 1 or 2, wherein said infrared radiation has a wavelength within the range 1 - 100 micrometers, preferably within the range 20 - 75 micrometers, even more preferably within the range 40 - 60 micrometers.

4. Method according to any one of claims 1-3, wherein said liquid (20) has a viscosity at 20°C within the range 0.5 mPa·s - 1.10⁶ mPa·s.

5. Method according to any one of claims 1-4, wherein said liquid (20) flows into said tubular conduit (12) at a volumetric flow rate within the range 0.1 1/min - 10 l/min, preferably within the range 1.0 1/min - 5.0 l/min.

6. Method according to any one of claims 1-5, wherein said liquid (20) is selected from: food liquid, cosmetic liquid, biological liquid, hydrocarbon liquid, liquid waste, heat transfer liquid.

7. Apparatus (100) for carrying out the method according to claim 1 comprising at least one treatment module (1) comprising:
- at least one tubular conduit (12) having at least one portion (14) transparent to infrared radiation;
- three sources (16a, 16b, 16c) of infrared radiation located in the vicinity of said portion (14) transparent to infrared radiation, said three sources (16a, 16b, 16c) being positioned along a direction parallel to the longitudinal axis of said tubular conduit (12), at a distance r from each other, and being configured to transmit at least an infrared radiation (18) on a liquid (20) flowing inside said tubular conduit (12);
wherein said distance r is within the range 1 cm - 50 cm;
wherein each of said sources (16a, 16b, 16c) extends for a length (L, L', L"), measured parallel to the longitudinal axis of said tubular conduit (12), within the range 10 cm - 100 cm.

8. Apparatus (100) according to claim 7, wherein said three sources (16a, 16b, 16c) develop predominantly linearly in a direction parallel to the longitudinal axis of said tubular conduit (12).

9. Apparatus (100) according to claim 7 or 8, wherein said distance r is within the range 5 cm - 30 cm.

10. Apparatus (100) according to any one of claims 7-9, wherein each of said sources (16a, 16b, 16c) extends for a length (L, L', L"), measured parallel to the longitudinal axis of said tubular conduit (12), within the range 15 cm - 80 cm, preferably within the range 20 cm - 60 cm.

11. Apparatus (100) according to any one of claims 7-10, wherein said tubular conduit (12) has a diameter within the range 0.1 cm - 2.0 cm, preferably within the range 0.2 cm - 1.2 cm.

12. Apparatus (100) according to any one of claims 7-11, wherein said treatment module (1) comprises a number of sources (16a, 16b, 16c) of infrared radiation within the range 2 - 10, preferably 2-5.

13. Apparatus (100) according to any one of claims 7-12, wherein each of said three sources (16a, 16b, 16c) has power within the range 1000 - 4000 W.

14. Apparatus (100) according to any one of claims 7-13, wherein said tubular conduit (12) is entirely made of a material transparent to infrared radiation.

15. Apparatus (100) according to claim 7-14, comprising two or more of said treatment modules (1a, 1b, 1c) connected to each other in parallel or in series.

## Patentansprüche

1. Verfahren zur Verminderung der mikrobiellen Belastung einer Flüssigkeit, das die folgenden Phasen umfasst:
a. Bereitstellen mindestens eines Behandlungsmoduls (1), das Folgendes umfasst:
- mindestens eine rohrförmige Leitung (12) mit mindestens einem für Infrarotstrahlung durchlässigen Abschnitt (14);
- drei Quellen (16a, 16b, 16c) für Infrarotstrahlung, die sich in der Nähe des für Infrarotstrahlung durchlässigen Abschnitts (14) befinden, wobei die drei Quellen (16a, 16b, 16c) entlang einer Richtung parallel zur Längsachse der rohrförmigen Leitung (12) in einem Abstand r voneinander positioniert und so konfiguriert sind, dass sie mindestens eine Infrarotstrahlung (18) übertragen;
b. Veranlassen, dass die Flüssigkeit (20) in die rohrförmige Leitung (12) fließt;
c. Aussetzen der Flüssigkeit (20), während sie im Inneren der rohrförmigen Leitung (12) fließt, der von den drei Quellen (16a, 16b, 16c) ausgesandten Infrarotstrahlung (18);
wobei die im Inneren der rohrförmigen Leitung fließende Flüssigkeit gemäß einer Bestrahlungssequenz behandelt wird, die Folgendes umfasst:
i) erste Bestrahlung der Flüssigkeit mit Infrarotstrahlung für eine Zeit im Bereich von 0,1 bis 5 Sekunden, was eine erste Erwärmungsphase darstellt;
ii) zweite Bestrahlung der Flüssigkeit mit Infrarotstrahlung für eine Zeit im Bereich von 0,2 bis 10 Sekunden, was eine zweite Erwärmungsphase darstellt;
ii) dritte Bestrahlung der Flüssigkeit mit Infrarotstrahlung für eine Zeit im Bereich von 1,5 bis 15 Sekunden, was eine dritte Erwärmungsphase darstellt;
wobei die Phasen i), ii) und iii) mit Perioden der Nicht-Belichtung der Flüssigkeit mit Infrarotstrahlung mit einer Dauer im Bereich von 0,1 - 7 Sekunden durchsetzt sind, welche Abkühlungsphasen darstellen.

2. Verfahren nach Anspruch 1, wobei sich die drei Quellen (16a, 16b, 16c) überwiegend linear in einer Richtung parallel zur Längsachse der rohrförmigen Leitung (12) entwickeln.

3. Verfahren nach Anspruch 1 oder 2, wobei die Infrarotstrahlung eine Wellenlänge im Bereich von 1 bis 100 Mikrometern, vorzugsweise im Bereich von 20 bis 75 Mikrometern, noch bevorzugter im Bereich von 40 bis 60 Mikrometern aufweist.

4. Verfahren nach einem der Ansprüche 1-3, wobei die Flüssigkeit (20) eine Viskosität bei 20°C im Bereich von 0,5 mPa-s - 1,106 mPa-s aufweist.

5. Verfahren nach einem der Ansprüche 1-4, wobei die Flüssigkeit (20) in die rohrförmige Leitung (12) mit einem Volumendurchsatz im Bereich von 0,1 1/min bis 10 1/min, vorzugsweise im Bereich von 1,0 1/min bis 5,0 1/min fließt.

6. Verfahren nach einem der Ansprüche 1-5, wobei die Flüssigkeit (20) ausgewählt ist aus: Lebensmittelflüssigkeit, kosmetischer Flüssigkeit, biologischer Flüssigkeit, Kohlenwasserstoffflüssigkeit, flüssigem Abfall, Wärmeübertragungsflüssigkeit.

7. Vorrichtung (100) zur Durchführung Verfahrens nach Anspruch 1, umfassend mindestens ein Behandlungsmodul (1), umfassend:
- mindestens eine rohrförmige Leitung (12) mit mindestens einem für Infrarotstrahlung durchlässigen Abschnitt (14);
- drei Quellen (16a, 16b, 16c) für Infrarotstrahlung, die sich in der Nähe des für Infrarotstrahlung durchlässigen Abschnitts (14) befinden, wobei die drei Quellen (16a, 16b, 16c) entlang einer Richtung parallel zur Längsachse der rohrförmigen Leitung (12) in einem Abstand r voneinander angeordnet und so konfiguriert sind, dass sie mindestens eine Infrarotstrahlung (18) auf eine in der rohrförmigen Leitung (12) fließende Flüssigkeit (20) übertragen;
wobei der Abstand r im Bereich von 1 cm bis 50 cm liegt;
wobei sich jede der Quellen (16a, 16b, 16c) über eine Länge (L, L', L"), gemessen parallel zur Längsachse der rohrförmigen Leitung (12), im Bereich von 10 cm bis 100 cm erstreckt.

8. Vorrichtung (100) nach Anspruch 7, wobei sich die drei Quellen (16a, 16b, 16c) überwiegend linear in einer Richtung parallel zur Längsachse der rohrförmigen Leitung (12) entwickeln.

9. Vorrichtung (100) nach Anspruch 7 oder 8, wobei der Abstand r im Bereich von 5 cm bis 30 cm liegt.

10. Vorrichtung (100) nach einem der Ansprüche 7-9, wobei sich jede der Quellen (16a, 16b, 16c) über eine Länge (L, L', L"), gemessen parallel zur Längsachse der rohrförmigen Leitung (12), im Bereich von 15 cm bis 80 cm, vorzugsweise im Bereich von 20 cm bis 60 cm, erstreckt.

11. Vorrichtung (100) nach einem der Ansprüche 7-10, wobei die rohrförmige Leitung (12) einen Durchmesser im Bereich von 0,1 cm bis 2,0 cm, vorzugsweise im Bereich von 0,2 cm bis 1,2 cm aufweist.

12. Vorrichtung (100) nach einem der Ansprüche 7-11, wobei das Behandlungsmodul (1) eine Anzahl von Infrarotstrahlungsquellen (16a, 16b, 16c) im Bereich von 2 bis 10, vorzugsweise 2 bis 5, aufweist.

13. Vorrichtung (100) nach einem der Ansprüche 7-12, wobei jede der drei Quellen (16a, 16b, 16c) eine Leistung im Bereich von 1000 - 4000 W aufweist.

14. Vorrichtung (100) nach einem der Ansprüche 7-13, wobei die rohrförmige Leitung (12) vollständig aus einem für Infrarotstrahlung durchlässigen Material besteht.

15. Vorrichtung (100) nach Anspruch 7-14, umfassend zwei oder mehr der Behandlungsmodule (1a, 1b, 1c), die parallel oder in Reihe miteinander geschaltet sind.

## Revendications

1. Procédé de réduction de la charge microbienne d'un liquide comprenant les phases suivantes :
a. fournir au moins un module de traitement (1) comprenant :
- au moins un conduit tubulaire (12) comportant au moins une partie (14) transparente au rayonnement infrarouge ;
- trois sources (16a, 16b, 16c) de rayonnement infrarouge situées à proximité de ladite partie (14) transparente au rayonnement infrarouge, lesdites trois sources (16a, 16b, 16c) étant positionnées selon une direction parallèle à l'axe longitudinal dudit conduit tubulaire (12), à une distance r les unes des autres, et étant configurées pour transmettre au moins un rayonnement infrarouge (18) ;
b. faire couler le liquide (20) dans ledit conduit tubulaire (12) ;
c. exposer le liquide (20) pendant qu'il s'écoule à l'intérieur du conduit tubulaire (12) au rayonnement infrarouge (18) émis par lesdites trois sources (16a, 16b, 16c) ;
dans lequel le liquide s'écoulant à l'intérieur du conduit tubulaire est traité selon une séquence d'irradiation comprenant :
i) une première exposition du liquide au rayonnement infrarouge pendant une durée comprise entre 0,1 et 5 secondes, ce qui constitue une première phase de chauffage ;
ii) une deuxième exposition du liquide au rayonnement infrarouge pendant une durée comprise entre 0,2 et 10 secondes, ce qui constitue une deuxième phase de chauffage ;
iii) une troisième exposition du liquide au rayonnement infrarouge pendant une durée comprise entre 1,5 et 15 secondes, ce qui constitue une troisième phase de chauffage ;
dans lequel lesdites phases i), ii) et iii) sont entrecoupées de périodes de non-exposition du liquide au rayonnement infrarouge d'une durée comprise entre 0,1 et 7 secondes, qui sont des phases de refroidissement.

2. Procédé selon la revendication 1, dans lequel lesdites trois sources (16a, 16b, 16c) se développent principalement de façon linéaire dans une direction parallèle à l'axe longitudinal dudit conduit tubulaire (12).

3. Procédé selon la revendication 1 ou 2, dans lequel ledit rayonnement infrarouge a une longueur d'onde comprise entre 1 et 100 micromètres, de préférence entre 20 et 75 micromètres, de préférence encore entre 40 et 60 micromètres.

4. Procédé selon l'une quelconque des revendications 1 à 3, dans lequel ledit liquide (20) a une viscosité à 20°C comprise entre 0,5 mPa s et 1,106 mPa s.

5. Procédé selon l'une quelconque des revendications 1 à 4, dans lequel ledit liquide (20) s'écoule dans ledit conduit tubulaire (12) à un débit volumétrique compris entre 0,1 1/min et 10 1/min, de préférence entre 1,0 1/min et 5,0 1/min.

6. Procédé selon l'une quelconque des revendications 1 à 5, dans lequel ledit liquide (20) est choisi parmi : les liquides alimentaires, les liquides cosmétiques, les liquides biologiques, les liquides d'hydrocarbures, les déchets liquides, les liquides de transfert de chaleur.

7. Appareil (100) pour la mise en œuvre du procédé selon la revendication 1 comprenant au moins un module de traitement (1) comprenant :
- au moins un conduit tubulaire (12) comportant au moins une partie (14) transparente au rayonnement infrarouge ;
- trois sources (16a, 16b, 16c) de rayonnement infrarouge situées à proximité de ladite partie (14) transparente au rayonnement infrarouge, lesdites trois sources (16a, 16b, 16c) étant positionnées selon une direction parallèle à l'axe longitudinal dudit conduit tubulaire (12), à une distance r les unes des autres, et étant configurées pour transmettre au moins un rayonnement infrarouge (18) sur un liquide (20) s'écoulant à l'intérieur dudit conduit tubulaire (12) ;
ladite distance r étant comprise entre 1 cm et 50 cm ;
dans lequel chacune desdites sources (16a, 16b, 16c) s'étend sur une longueur (L, L', L"), mesurée parallèlement à l'axe longitudinal dudit conduit tubulaire (12), comprise entre 10 cm et 100 cm.

8. Appareil (100) selon la revendication 7, dans lequel lesdites trois sources (16a, 16b, 16c) se développent principalement de façon linéaire dans une direction parallèle à l'axe longitudinal dudit conduit tubulaire (12).

9. Appareil (100) selon la revendication 7 ou 8, dans lequel ladite distance r est comprise entre 5 cm et 30 cm.

10. Appareil (100) selon l'une quelconque des revendications 7 à 9, dans lequel chacune desdites sources (16a, 16b, 16c) s'étend sur une longueur (L, L', L"), mesurée parallèlement à l'axe longitudinal dudit conduit tubulaire (12), comprise entre 15 cm et 80 cm, de préférence entre 20 cm et 60 cm.

11. Appareil (100) selon l'une quelconque des revendications 7 à 10, dans lequel ledit conduit tubulaire (12) a un diamètre compris entre 0,1 cm et 2,0 cm, de préférence entre 0,2 cm et 1,2 cm.

12. Appareil (100) selon l'une quelconque des revendications 7 à 11, dans lequel ledit module de traitement (1) comprend un nombre de sources (16a, 16b, 16c) de rayonnement infrarouge compris entre 2 et 10, de préférence entre 2 et 5.

13. Appareil (100) selon l'une quelconque des revendications 7 à 12, dans lequel chacune desdites trois sources (16a, 16b, 16c) a une puissance comprise entre 1000 et 4000 W.

14. Appareil (100) selon l'une quelconque des revendications 7 à 13, dans lequel ledit conduit tubulaire (12) est entièrement constitué d'un matériau transparent au rayonnement infrarouge.

15. Appareil (100) selon les revendications 7 à 14, comprenant au moins deux desdits modules de traitement (1a, 1b, 1c) reliés les uns aux autres en parallèle ou en série.
